# EUROPEAN PATENT SPECIFICATION

(11) **EP 3 397 058 B1**
(45) Date of publication and mention of the grant of the patent: **21.10.2020**
(21) Application number: 16882412.6
(22) Date of filing: 22.12.2016
(51) Int. Cl.: A01P 3/00, A01N 43/30, A01N 43/40, C07D 239/52, C07D 249/08, C07D 249/12, C07D 405/12, C07D 405/00, C07D 405/02, A01N 25/00

(54) **FUNGAL CONTROL OF WHITE MOLD**
PILZBEKÄMPFUNG VON WEISSSCHIMMEL
RÉGULATION FONGIQUE DE MOISISSURE BLANCHE

(30) Priority: 30.12.2015 US 201562273398 P
(43) Date of publication of application: 07.11.2018
(73) Proprietor: Dow Agrosciences LLC, Indianapolis, IN 46268 (US)
(72) Inventor: CORREA DA SILVA, Olavo, Indianapolis IN 46268 (US); KEMMITT, Greg, Indianapolis IN 46268 (US); BERNHARD, Hans U., Indianapolis IN 46268 (US); CAILLIAU, Mathilde M., Indianapolis IN 46268 (US)
(74) Representative: f & e patent
(86) International application number: PCT/US2016/068219
(87) International publication number: WO 2017/116951

(56) References cited:
- US-A1- 2011 003 869
- US-A1- 2013 296 371
- US-A1- 2014 187 590
- US-A1- 2015 282 490

## Description

### FIELD OF THE INVENTION

This disclosure concerns a fungicidal composition containing a compound of Formula I, (3*S*,6*S*,7*R*,8*R*)-8-benzyl-3-(3-((isobutyryloxy)methoxy)-4-methoxypicolinamido)-6-methyl-4,9-dioxo-1,5-dioxonan-7-yl isobutyrate for use on white mold. Additionally, this disclosure concerns a fungicidal composition containing (a) a compound of Formula I, (3*S*,6*S*,7*R*,8*R*)-8-benzyl-3-(3-((isobutyryloxy)methoxy)-4-methoxypicolinamido)-6-methyl-4,9-dioxo-1,5-dioxonan-7-yl isobutyrate and (b) at least one fungicide selected from the group consisting of azoxystrobin, prothioconazole and tebuconazole for control of white mold.

### BACKGROUND AND SUMMARY

Fungicides are compounds, of natural or synthetic origin, which act to protect plants against damage caused by fungi. Current methods of agriculture rely heavily on the use of fungicides. In fact, some crops cannot be grown usefully without the use of fungicides. Using fungicides allows a grower to increase the yield and the quality of the crop, and consequently, increase the value of the crop. In most situations, the increase in value of the crop is worth at least three times the cost of the use of the fungicide.

US2011/003869 discloses a composition comprising a succinate dehydrogenase inhibitor and at least one triazole fungicide for controlling *Sclerotinia ssp..* US2015/282490 discloses a composition comprising at least one biological control agent selected from the group consisting of a *Paecilomyces lilacinus* strain and *Coniothyrium minitans* and at least one fungicide selected from the group consisting of inhibitors of the ergosterol biosynthesis for controlling *Sclerotinia ssp..*

However, no one fungicide is useful in all situations and repeated usage of a single fungicide frequently leads to the development of resistance to that and related fungicides. Consequently, research is being conducted to produce fungicides and combinations of fungicides that are safer, that have better performance, that require lower dosages, that are easier to use, and that cost less.

It is an object of this disclosure to provide compositions comprising fungicidal compounds. It is a further object of this disclosure to provide processes that use these compositions. The compositions are capable of preventing or curing, or both, diseases caused by the *Ascomycete* pathogen white mold. In accordance with this disclosure, compositions are provided along with methods for their use.

### Detailed Description

The present disclosure concerns a fungicidal composition comprising an fungicidally effective amount of a compound of Formula I, (3*S*,6*S*,7*R*,8*R*)-8-benzyl-3-(3-((isobutyryloxy)methoxy)-4-methoxypicolinamido)-6-methyl-4,9-dioxo-1,5-dioxonan-7-yl isobutyrate for use on white mold. Additionally, this disclosure concerns a fungicidal composition containing (a) a compound of Formula I, (3*S*,6*S*,7*R*,8*R*)-8-benzyl-3-(3-((isobutyryloxy)methoxy)-4-methoxypicolinamido)-6-methyl-4,9-dioxo-1,5-dioxonan-7-yl isobutyrate and (b) at least one fungicide selected from the group consisting of azoxystrobin, prothioconazole and tebuconazole for control of white mold.

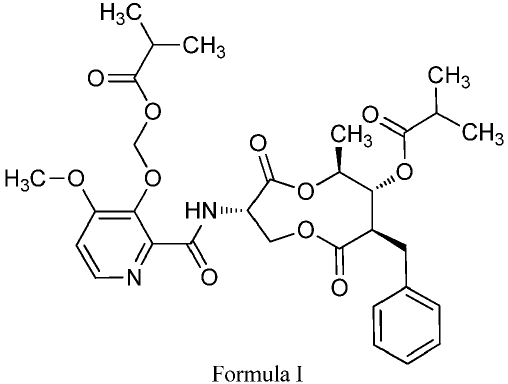

As used herein, azoxystrobin is the common name for methyl (2*E*)-2-{2-[6-(2-cyanophenoxy)pyrimidin-4-yloxy]phenyl}-3-methoxyacrylate and possesses the following structure:

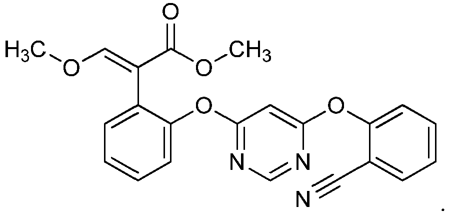

Its fungicidal activity is exemplified in The e-Pesticide Manual, Version 5.2, 2011**.** Exemplary uses of azoxystrobin include, but are not limited to, control of the following pathogens at the following rates (100 to 375 g/ha): *Erysiphe graminis, Puccinia* spp., *Leptosphaeria nodorum, Septoria tritici* and *Pyrenophora teres* on temperate cereals; *Pyricularia oryzae* and *Rhizoctonia solani* on rice; *Plasmopara viticola* and *Uncinula necator* on vines; *Sphaerotheca fuliginea* and *Pseudoperonospora cubensis* on cucurbitaceae; *Phytophthora infestans* and *Alternaria solani* on potato and tomato; *Mycosphaerella arachidis, Rhizoctonia solani* and *Sclerotium rolfsii* on peanut; *Monilinia* spp. and *Cladosporium carpophilum* on peach; *Pythium* spp. and *Rhizoctonia solani* on turf; *Mycosphaerella* spp. on banana; *Cladosporium caryigenum* on pecan; *Elsinoë fawcettii, Colletotrichum* spp. and *Guignardia citricarpa* on citrus; *Colletotrichum* spp. and *Hemileia vastatrix* on coffee.

As used herein, tebuconazole is the common name for α-[2-(4-chlorophenyl)ethyl]-α-(1,1-dimethylethyl)-1*H*-1,2,4-triazole-1-ethanol and possesses the following structure:

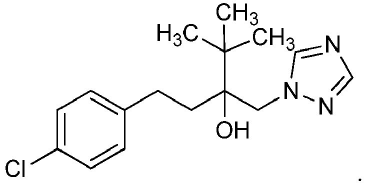

Its fungicidal activity is described in The Pesticide Manual, Fourteenth Edition, 2006. Tebuconazole is a commercial fungicide used to control fungal diseases in a variety of agricultural crops, particularly cereals, including wheat, barley, and canola, as well as peanuts, oilseed rape, grapes, pome fruit, stone fruit, and bananas.As used herein, picoxystrobin is the common name for methyl (*E*)-3-methoxy-2-[2-(6-trifluoromethyl-2-pyridyloxymethyl)phenyl]acrylate and possesses the following structure:

As used herein, prothioconazole is the common name 2-[(2*RS*)-2-(1-chlorocyclopropyl)-3-(2-chlorophenyl)-2-hydroxypropyl]-2*H*-1,2,4-triazole-3(4*H*)-thione and possesses the following structure:

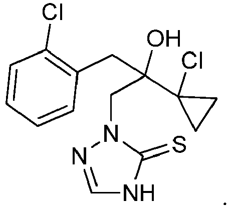

Its fungicidal activity is described in The Pesticide Manual, Fifteenth Edition, 2009. Prothioconazole provides control of diseases such as eyespot (*Pseudocercosporella herpotrichoides*), Fusarium ear blight (*Fusarium* spp., *Microdochium nivale*), leaf blotch diseases (*Septoria tritici, Leptosphaeria nodorum, Pyrenophora* spp., *Rhynchosporium secalis,* etc.), rust (*Puccinia* spp.) and powdery mildew (*Blumeria graminis*), by foliar application, in wheat, barley and other crops.

The components of the composition of the present disclosure can be applied either separately or as part of a multipart fungicidal system.

The mixture of the present disclosure can be applied in conjunction with one or more other fungicides to control a wider variety of undesirable diseases. When used in conjunction with other fungicide(s), the presently claimed compounds may be formulated with the other fungicide(s), tank mixed with the other fungicide(s) or applied sequentially with the other fungicide(s). Such other fungicides may include 2-(thiocyanatomethylthio)-benzothiazole, 2-phenylphenol, 8-hydroxyquinoline sulfate, ametoctradin, amisulbrom, antimycin, *Ampelomyces quisqualis,* azaconazole, azoxystrobin, *Bacillus subtilis, Bacillus subtilis* strain QST713, benalaxyl, benomyl, benthiavalicarb-isopropyl, benzovindiflupyr benzylaminobenzene-sulfonate (BABS) salt, bicarbonates, biphenyl, bismerthiazol, bitertanol, bixafen, blasticidin-S, borax, Bordeaux mixture, boscalid, bromuconazole, bupirimate, calcium polysulfide, captafol, captan, carbendazim, carboxin, carpropamid, carvone, chlazafenone, chloroneb, chlorothalonil, chlozolinate, *Coniothyrium minitans,* copper hydroxide, copper octanoate, copper oxychloride, copper sulfate, copper sulfate (tribasic), cuprous oxide, cyazofamid, cyflufenamid, cymoxanil, cyproconazole, cyprodinil, dazomet, debacarb, diammonium ethylenebis-(dithiocarbamate), dichlofluanid, dichlorophen, diclocymet, diclomezine, dichloran, diethofencarb, difenoconazole, difenzoquat ion, diflumetorim, dimethomorph, dimoxystrobin, diniconazole, diniconazole-M, dinobuton, dinocap, diphenylamine, dithianon, dodemorph, dodemorph acetate, dodine, dodine free base, edifenphos, enestrobin, enestroburin, epoxiconazole, ethaboxam, ethoxyquin, etridiazole, famoxadone, fenamidone, fenarimol, fenbuconazole, fenfuram, fenhexamid, fenoxanil, fenpiclonil, fenpropidin, fenpropimorph, fenpyrazamine, fentin, fentin acetate, fentin hydroxide, ferbam, ferimzone, fluazinam, fludioxonil, flumorph, fluopicolide, fluopyram, fluoroimide, fluoxastrobin, fluquinconazole, flusilazole, flusulfamide, flutianil, flutolanil, flutriafol, fluxapyroxad, folpet, formaldehyde, fosetyl, fosetyl-aluminium, fuberidazole, furalaxyl, furametpyr, guazatine, guazatine acetates, GY-81, hexachlorobenzene, hexaconazole, hymexazol, imazalil, imazalil sulfate, imibenconazole, iminoctadine, iminoctadine triacetate, iminoctadine tris(albesilate), iodocarb, ipconazole, ipfenpyrazolone, iprobenfos, iprodione, iprovalicarb, isoprothiolane, isopyrazam, isotianil, kasugamycin, kasugamycin hydrochloride hydrate, kresoxim-methyl, laminarin, mancopper, mancozeb, mandipropamid, maneb, mefenoxam, mepanipyrim, mepronil, meptyl-dinocap, mercuric chloride, mercuric oxide, mercurous chloride, metalaxyl, metalaxyl-M, metam, metam-ammonium, metam-potassium, metam-sodium, metconazole, methasulfocarb, methyl iodide, methyl isothiocyanate, metiram, metominostrobin, metrafenone, mildiomycin, myclobutanil, nabam, nitrothal-isopropyl, nuarimol, octhilinone, ofurace, oleic acid (fatty acids), orysastrobin, oxadixyl, oxine-copper, oxpoconazole fumarate, oxycarboxin, pefurazoate, penconazole, pencycuron, penflufen, pentachlorophenol, pentachlorophenyl laurate, penthiopyrad, phenylmercury acetate, phosphonic acid, phthalide, picoxystrobin, polyoxin B, polyoxins, polyoxorim, potassium bicarbonate, potassium hydroxyquinoline sulfate, probenazole, prochloraz, procymidone, propamocarb, propamocarb hydrochloride, propiconazole, propineb, proquinazid, prothioconazole, pyraclostrobin, pyrametostrobin, pyraoxystrobin, pyrazophos, pyribencarb, pyributicarb, pyrifenox, pyrimethanil, pyriofenone, pyroquilon, quinoclamine, quinoxyfen, quintozene, *Reynoutria sachalinensis* extract, sedaxane, silthiofam, simeconazole, sodium 2-phenylphenoxide, sodium bicarbonate, sodium pentachlorophenoxide, spiroxamine, sulfur, SYP-Z048, tar oils, tebuconazole, tebufloquin, tecnazene, tetraconazole, thiabendazole, thifluzamide, thiophanate-methyl, thiram, tiadinil, tolclofos-methyl, tolylfluanid, triadimefon, triadimenol, triazoxide, tricyclazole, tridemorph, trifloxystrobin, triflumizole, triforine, triticonazole, validamycin, valifenalate, valiphenal, vinclozolin, zineb, ziram, zoxamide, *Candida oleophila, Fusarium oxysporum, Gliocladium* spp., *Phlebiopsis gigantea, Streptomyces griseoviridis, Trichoderma* spp., (*RS*)*-N*-(3,5-dichlorophenyl)-2-(methoxymethyl)-succinimide, 1,2-dichloropropane, 1,3-dichloro-1,1,3,3-tetrafluoroacetone hydrate, 1-chloro-2,4-dinitronaphthalene, 1-chloro-2-nitropropane, 2-(2-heptadecyl-2-imidazolin-1-yl)ethanol, 2,3-dihydro-5-phenyl-1,4-dithi-ine 1,1,4,4-tetraoxide, 2-methoxyethylmercury acetate, 2-methoxyethylmercury chloride, 2-methoxyethylmercury silicate, 3-(4-chlorophenyl)-5-methylrhodanine, 4-(2-nitroprop-1-enyl)phenyl thiocyanateme, ampropylfos, anilazine, azithiram, barium polysulfide, Bayer 32394, benodanil, benquinox, bentaluron, benzamacril; benzamacril-isobutyl, benzamorf, binapacryl, bis(methylmercury) sulfate, bis(tributyltin) oxide, buthiobate, cadmium calcium copper zinc chromate sulfate, carbamorph, CECA, chlobenthiazone, chloraniformethan, chlorfenazole, chlorquinox, climbazole, copper bis(3-phenylsalicylate), copper zinc chromate, cufraneb, cupric hydrazinium sulfate, cuprobam, cyclafuramid, cypendazole, cyprofuram, decafentin, dichlone, dichlozoline, diclobutrazol, dimethirimol, dinocton, dinosulfon, dinoterbon, dipyrithione, ditalimfos, dodicin, drazoxolon, EBP, ESBP, etaconazole, etem, ethirim, fenaminosulf, fenapanil, fenitropan, fluotrimazole, furcarbanil, furconazole, furconazole-cis, furmecyclox, furophanate, glyodine, griseofulvin, halacrinate, Hercules 3944, hexylthiofos, ICIA0858, isopamphos, isovaledione, mebenil, mecarbinzid, metazoxolon, methfuroxam, methylmercury dicyandiamide, metsulfovax, milneb, mucochloric anhydride, myclozolin, *N-*3,5-dichlorophenyl-succinimide, *N*-3-nitrophenylitaconimide, natamycin, *N*-ethylmercurio-4-toluenesulfonanilide, nickel bis(dimethyldithiocarbamate), OCH, phenylmercury dimethyldithiocarbamate, phenylmercury nitrate, phosdiphen, prothiocarb; prothiocarb hydrochloride, pyracarbolid, pyridinitril, pyroxychlor, pyroxyfur, quinacetol; quinacetol sulfate, quinazamid, quinconazole, rabenzazole, salicylanilide, SSF-109, sultropen, tecoram, thiadifluor, thicyofen, thiochlorfenphim, thiophanate, thioquinox, tioxymid, triamiphos, triarimol, triazbutil, trichlamide, urbacid, zarilamid, and any combinations thereof.

The compositions of the present disclosure are preferably applied in the form of a formulation comprising a composition of (a) a compound of Formula I and/or (b) at least one fungicide selected from azoxystrobin, prothioconazole and tebuconazole, together with a phytologically acceptable carrier.

Concentrated formulations can be dispersed in water, or another liquid, for application, or formulations can be dust-like or granular, which can then be applied without further treatment. The formulations are prepared according to procedures which are conventional in the agricultural chemical art, but which are novel and important because of the presence therein of a composition.

The formulations that are applied most often are aqueous suspensions or emulsions. Either such water-soluble, water-suspendable, or emulsifiable formulations are solids, usually known as wettable powders, or liquids, usually known as emulsifiable concentrates, aqueous suspensions, or suspension concentrates. The present disclosure contemplates all vehicles by which the compositions can be formulated for delivery and use as a fungicide.

As will be readily appreciated, any material to which these compositions can be added may be used, provided they yield the desired utility without significant interference with the activity of these compositions as antifungal agents.

Wettable powders, which may be compacted to form water-dispersible granules, comprise an intimate mixture of the composition, a carrier and agriculturally acceptable surfactants. The concentration of the composition in the wettable powder is usually from about 10% to about 90% by weight, more preferably about 25% to about 75% by weight, based on the total weight of the formulation. In the preparation of wettable powder formulations, the composition can be compounded with any of the finely divided solids, such as prophyllite, talc, chalk, gypsum, Fuller's earth, bentonite, attapulgite, starch, casein, gluten, montmorillonite clays, diatomaceous earths, purified silicates or the like. In such operations, the finely divided carrier is ground or mixed with the composition in a volatile organic solvent. Effective surfactants, comprising from about 0.5% to about 10% by weight of the wettable powder, include sulfonated lignins, naphthalenesulfonates, alkylbenzenesulfonates, alkyl sulfates, and non-ionic surfactants, such as ethylene oxide adducts of alkyl phenols.

Emulsifiable concentrates of the composition comprise a convenient concentration, such as from about 10% to about 50% by weight, in a suitable liquid, based on the total weight of the emulsifiable concentrate formulation. The components of the compositions, jointly or separately, are dissolved in a carrier, which is either a water-miscible solvent or a mixture of water-immiscible organic solvents, and emulsifiers. The concentrates may be diluted with water and oil to form spray mixtures in the form of oil-in-water emulsions. Useful organic solvents include aromatics, especially the high-boiling naphthalenic and olefinic portions of petroleum such as heavy aromatic naphtha. Other organic solvents may also be used, such as, for example, terpenic solvents, including rosin derivatives, aliphatic ketones, such as cyclohexanone, and complex alcohols, such as 2-ethoxyethanol.

Emulsifiers which can be advantageously employed herein can be readily determined by those skilled in the art and include various nonionic, anionic, cationic and amphoteric emulsifiers, or a blend of two or more emulsifiers. Examples of nonionic emulsifiers useful in preparing the emulsifiable concentrates include the polyalkylene glycol ethers and condensation products of alkyl and aryl phenols, aliphatic alcohols, aliphatic amines or fatty acids with ethylene oxide, propylene oxides such as the ethoxylated alkyl phenols and carboxylic esters solubilized with the polyol or polyoxyalkylene. Cationic emulsifiers include quaternary ammonium compounds and fatty amine salts. Anionic emulsifiers include the oil-soluble salts (e.g., calcium) of alkylaryl sulfonic acids, oil-soluble salts or sulfated polyglycol ethers and appropriate salts of phosphated polyglycol ether.

Representative organic liquids which can be employed in preparing the emulsifiable concentrates of the present disclosure are the aromatic liquids such as xylene, propyl benzene fractions, or mixed naphthalene fractions, mineral oils, substituted aromatic organic liquids such as dioctyl phthalate, kerosene, dialkyl amides of various fatty acids, particularly the dimethyl amides of fatty glycols and glycol derivatives such as the n-butyl ether, ethyl ether or methyl ether of diethylene glycol, and the methyl ether of triethylene glycol. Mixtures of two or more organic liquids are also often suitably employed in the preparation of the emulsifiable concentrate. The preferred organic liquids are xylene, and propyl benzene fractions, with xylene being most preferred. The surface-active dispersing agents are usually employed in liquid formulations and in the amount of from 0.1 to 20 percent by weight of the combined weight of the dispersing agent with the compositions. The formulations can also contain other compatible additives, for example, plant growth regulators and other biologically active compounds used in agriculture.

Aqueous suspensions comprise suspensions of one or more water-insoluble compounds, dispersed in an aqueous vehicle at a concentration in the range from about 5% to about 70% by weight, based on the total weight of the aqueous suspension formulation. Suspensions are prepared by finely grinding the components of the combination either together or separately, and vigorously mixing the ground material into a vehicle comprised of water and surfactants chosen from the same types discussed above. Other ingredients, such as inorganic salts and synthetic or natural gums, may also be added to increase the density and viscosity of the aqueous vehicle. It is often most effective to grind and mix at the same time by preparing the aqueous mixture and homogenizing it in an implement such as a sand mill, ball mill, or piston-type homogenizer.

The composition may also be applied as a granular formulation, which is particularly useful for applications to the soil. Granular formulations usually contain from about 0.5% to about 10% by weight of the compounds, based on the total weight of the granular formulation, dispersed in a carrier which consists entirely or in large part of coarsely divided attapulgite, bentonite, diatomite, clay or a similar inexpensive substance. Such formulations are usually prepared by dissolving the composition in a suitable solvent and applying it to a granular carrier which has been preformed to the appropriate particle size, in the range of from about 0.5 to about 3 mm. Such formulations may also be prepared by making a dough or paste of the carrier and the composition, and crushing and drying to obtain the desired granular particle.

Dusts containing the composition are prepared simply by intimately mixing the composition in powdered form with a suitable dusty agricultural carrier, such as, for example, kaolin clay, ground volcanic rock, and the like. Dusts can suitably contain from about 1% to about 10% by weight of the composition/carrier combination.

The formulations may contain agriculturally acceptable adjuvant surfactants to enhance deposition, wetting and penetration of the composition onto the target crop and organism. These adjuvant surfactants may optionally be employed as a component of the formulation or as a tank mix. The amount of adjuvant surfactant will vary from 0.01 percent to 1.0 percent volume/volume (v/v) based on a spray-volume of water, preferably 0.05 to 0.5 percent. Suitable adjuvant surfactants include ethoxylated nonyl phenols, ethoxylated synthetic or natural alcohols, salts of the esters or sulfosuccinic acids, ethoxylated organosilicones, ethoxylated fatty amines and blends of surfactants with mineral or vegetable oils.

The formulations may optionally include combinations that can comprise at least 1% by weight of one or more of the compositions with another pesticidal compound. Such additional pesticidal compounds may be fungicides, insecticides, nematocides, miticides, arthropodicides, bactericides or combinations thereof that are compatible with the compositions of the present disclosure in the medium selected for application, and not antagonistic to the activity of the present compounds. Accordingly, in such embodiments the other pesticidal compound is employed as a supplemental toxicant for the same or for a different pesticidal use. The pesticidal compound and the composition can generally be mixed together in a weight ratio of from 1:100 to 100:1.

The present disclosure includes within its scope methods for the control or prevention of fungal attack. These methods comprise applying to the locus of the fungus, or to a locus in which the infestation is to be prevented (for example applying to wheat or barley plants), a fungicidally effective amount of the composition. The composition is suitable for treatment of various plants at fungicidal levels, while exhibiting low phytotoxicity. The composition is useful in a protectant or eradicant fashion. The composition is applied by any of a variety of known techniques, either as the composition or as a formulation comprising the composition. For example, the compositions may be applied to the roots, seeds or foliage of plants for the control of various fungi, without damaging the commercial value of the plants. The composition is applied in the form of any of the generally used formulation types, for example, as solutions, dusts, wettable powders, flowable concentrates, or emulsifiable concentrates. These materials are conveniently applied in various known fashions.

The composition has been found to have significant fungicidal effect, particularly for agricultural use. The composition is particularly effective for use with agricultural crops and horticultural plants, or with wood, paint, leather or carpet backing.

In particular, the composition is effective in controlling a variety of undesirable fungi that infect useful plant crops. The composition may be used against a variety of *Ascomycete* and *Basidiomycete* fungi, including for example the following representative fungi species: leaf blotch of wheat (*Mycosphaerella graminicola;* anamorph: *Septoria tritici;* Bayer code SEPTTR); glume blotch of wheat (*Leptosphaeria nodorum;* Bayer code LEPTNO; anamorph: *Stagonospora nodorum*); spot blotch of barley (*Cochliobolus sativum;* Bayer code COCHSA; anamorph: *Helminthosporium sativum*); leaf spot of sugar beets (*Cercospora beticola;* Bayer code CERCBE); leaf spot of peanut (*Mycosphaerella arachidis;* Bayer code MYCOAR; anamorph: *Cercospora arachidicola*); cucumber anthracnose (*Glomerella lagenarium;* anamorph: *Colletotrichum lagenarium;* Bayer code COLLLA); black sigatoka disease of banana (*Mycosphaerella fijiensis;* Bayer code MYCOFI) and white mold (*Sclerotinia sclerotiorum;* Bayer code: SCLESC). It will be understood by those in the art that the efficacy of the compositions for one or more of the foregoing fungi establishes the general utility of the compositions as fungicides.

The compositions have a broad range of efficacy as a fungicide. The exact amount of the composition to be applied is dependent not only on the relative amounts of the components, but also on the particular action desired, the fungal species to be controlled, and the stage of growth thereof, as well as the part of the plant or other product to be contacted with the composition. Thus, formulations containing the composition may not be equally effective at similar concentrations or against the same fungal species.

The compositions are effective in use with plants in a disease-inhibiting and phytologically acceptable amount. The term "disease-inhibiting and phytologically acceptable amount" refers to an amount of the composition that kills or inhibits the plant disease for which control is desired, but is not significantly toxic to the plant. The exact concentration of composition required varies with the fungal disease to be controlled, the type of formulation employed, the method of application, the particular plant species, climate conditions, and the like.

The present compositions can be applied to fungi or their locus by the use of conventional ground sprayers, granule applicators, and by other conventional means known to those skilled in the art.

The following examples are provided to further illustrate the disclosure. They are not meant to be construed as limiting the disclosure.

### Examples

### Evaluation of Inhibition of Mycelial Growth by Fungicides (Sclerotinia sclerotiorum; Bayer code: SCLESC):

The growth inhibition of the fungus (*Sclerotinia sclerotiorum*) was assessed in Petri dishes containing the growth medium + fungicide at different concentrations. The plates were incubated in a growth chamber (BOD) at 22 °C with a photoperiod of 12 hours (h) for 8 days (d). Growth measurements were made across the diameter of the colony beginning 2 d after inoculation. The number of sclerotia were counted. A completely randomized design with three replicates was used.

Treatments consisted of fungicide compounds including Formula I, fluazinam, thiophanate-methyl (reference example), and procymidone. Data are presented in Tables 1 - 2.

### Evaluation of Inhibition of Sclerotinia sclerotiorum on Detached Soybean Leaves:

Detached leaves of soybean were immersed for 3 seconds (s) in fungicide solutions at different concentrations and placed in Petri dishes. After immersion the leaves were inoculated with a 1 centimeter (cm) disk of mycelium. Symptoms were assessed for percent disease severity. Three leaves per plate with three replicates in a completely randomized design.

Treatments consisted of fungicide compounds including Formula I, fluazinam, thiophanate-methyl (reference example), and procymidone. Data are presented in Table 3.

### Evaluation of Incidence of Sclerotinia sclerotiorum at R7 Growth Stage on Dry Bean:

A field trial in dry bean was carried out in the state of Sao Paulo, Brazil. Two applications of the fungicides were done at growth stages R5 (first bloom) and R6 (full bloom) by using a carbon dioxide (CO₂) backpack sprayer at an application rate of 600 liters per hectare (L/ha). The incidence of white mold was assessed at growth stages R6 and R7.

Treatments consisted of fungicide compounds including Formula I, fluazinam, thiophanate-methyl (reference example), and procymidone. Data are presented in Table 4.

### Evaluation of Incidence of Sclerotinia sclerotiorum and Yield of Oilseed Rape at B65 to B67 Growth Stages

Three field trials in oilseed rape were carried out in Niedersachsen, Sachsen and Mecklenburg-Vorpommern, Germany and one in Bourgogne, France. A single application of each fungicide was done at growth stages B65 to B67. The incidence of white mold and yield of oilseed rape were assessed.

Treatments consisted of fungicide compounds including Formula I, azoxystrobin, tebuconazole, prothioconazole, boscalid (reference example), and isopyrazam + cyproconazole (reference example). Data are presented in Tables 5 - 6.

**Table 1: Percent (%) Inhibition of Mycelial Growth of Sclerotinia sclerotiorum**

| Treatment | Concentration (ppm)* | | | |
|---|---|---|---|---|
| | 0.1 | 1 | 10 | 100 |
| Fluazinam | 59.7 | 71.64 | 82.61 | 100 |
| Thiophanate-Methyl | 0 | 32.2 | 78.32 | 100 |
| Procymidone | 10 | 24.48 | 68.21 | 100 |
| Compound I | 28.33 | 72.39 | 80.74 | 91.04 |

| | | | | |
|---|---|---|---|---|
| *ppm = Parts per million | | | | |

**Table 2: Number of Sclerotinia Formed per Colony**

| Treatment | Concentration (ppm)* | | | |
|---|---|---|---|---|
| | 0.1 | 1 | 10 | 100 |
| Fluazinam | 5.7 | 4.3 | 0 | 0 |
| Thiophanate-Methyl | 19.25 | 17.56 | 0 | 0 |
| Procymidone | 18.67 | 16.2 | 1.67 | 0 |
| Compound I | 3.33 | 2.33 | 1.67 | 1.33 |

| | | | | |
|---|---|---|---|---|
| *ppm = Parts per million | | | | |

**Table 3: Percent (%) Inhibition of Sclerotinia sclerotiorum on Detached Soybean Leaves**

| Treatment | Concentration (ppm)* | | | |
|---|---|---|---|---|
| | 0.1 | 1 | 10 | 100 |
| Fluazinam | 25.86 | 53.45 | 66.09 | 100 |
| Thiophanate-Methyl | 20.69 | 43.11 | 87.36 | 100 |
| Procymidone | 19.54 | 59.77 | 85.06 | 100 |
| Compound I | 8.63 | 66.09 | 92.53 | 100 |

| | | | | |
|---|---|---|---|---|
| *ppm = Parts per million | | | | |

**Table 4: Percent (%) Incidence of Sclerotinia sclerotiorum at R7 Growth Stage on Dry Bean**

| Treatment | Application Rate g ai/ha* | Percent (%) Incidence |
|---|---|---|
| Compound I | 120 | 3.5 |
| Compound I | 240 | 1 |
| Compound I + Fluazinam | 120 + 250 | 1 |
| Compound I + Procymidone | 120 + 250 | 0.8 |
| Fluazinam | 500 | 0.8 |
| Untreated | - | 12.5 |

| | | |
|---|---|---|
| *g ai/ha = grams active ingrediant per hectare | | |

**Table 5: Percent (%) Incidence of Sclerotinia sclerotiorum at B65-B67 Growth Stage in Oilseed Rape**

| Treatment | Application Rate g ai/ha* | Percent (%) Incidence |
|---|---|---|
| Compound I | 100 | 24 |
| Compound I | 130 | 24 |
| Compound I | 150 | 19 |
| Compound I | 200 | 14 |
| Compound I + Azoxystrobin | 130 + 200 | 3 |
| Compound I + Tebuconazole | 130 + 200 | 9 |
| Compound I + Prothioconazole | 100 + 150 | 22 |
| Compound I + Prothioconazole | 115 + 173 | 23 |
| Prothioconazole | 150 | 31 |
| Prothioconazole | 173 | 20 |
| Boscalid | 250 | 4 |
| Isopyrazam + Cyproconazole | 125 + 80 | 19 |
| Untreated | - | 55 |

| | | |
|---|---|---|
| *g ai/ha = grams active ingrediant per hectare | | |

**Table 6: Effect of Fungicide Treatment on Yield of Oilseed Rape (T/ha)**

| Treatment | Application Rate g ai/ha* | Yield (T/ha) |
|---|---|---|
| Compound I | 100 | 4.22 |
| Compound I | 130 | 4.29 |
| Compound I | 150 | 4.23 |
| Compound I | 200 | 4.48 |
| Compound I + Azoxystrobin | 130 + 200 | 4.4 |
| Compound I + Tebuconazole | 130 + 200 | 4.42 |
| Compound I + Prothioconazole | 100 + 150 | 4.35 |
| Compound I + Prothioconazole | 115 + 173 | 4.43 |
| Prothioconazole | 150 | 4.4 |
| Prothioconazole | 173 | 4.49 |
| Boscalid | 250 | 4.34 |
| Isopyrazam + Cyproconazole | 125 + 80 | 4.53 |
| Untreated | - | 4.08 |

| | | |
|---|---|---|
| *g ai/ha = grams active ingrediant per hectare **T/ha = ton per hectare | | |

## Claims

1. A method for the control and prevention of white mold on a plant, the method comprising: applying a fungicidally effective amount of a formulation including a compound of Formula I, wherein said effective amount is applied to at least one of the plant, an area adjacent to the plant, soil adapted to support growth of the plant, a root of the plant, foliage of the plant, and a seed adapted to produce the plant.

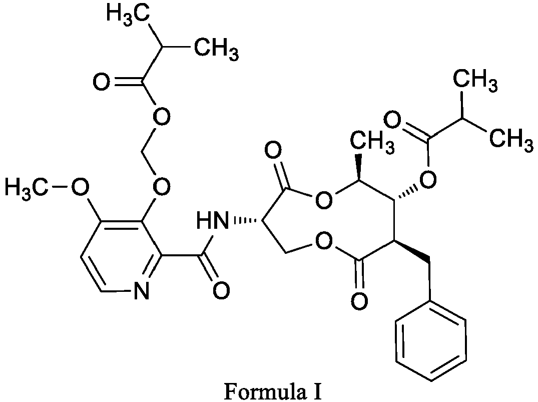

2. The method of claim 1, wherein the formulation further comprises an agriculturally acceptable adjuvant or carrier.

3. The method of claim 1 or claim 2, wherein the formulation further comprises azoxystrobin.

4. The method of claim 1 or claim 2, wherein the formulation further comprises tebuconazole.

5. The method of claim 1 or claim 2, wherein the formulation further comprises prothioconazole.

6. The method of claim 1 comprising: applying a fungicidally effective amount of the compound of Formula I and at least one fungicide selected from the group consisting of azoxystrobin, tebuconazole, and prothioconazole.

## Patentansprüche

1. Ein Verfahren zur Bekämpfung und Vorbeugung von weißem Schimmel auf einer Pflanze, das Verfahren umfassend: Anwenden einer fungizid wirksamen Menge einer Formulierung, die eine Verbindung der Formel I beinhaltet, wobei diese wirksame Menge auf zumindest eines von der Pflanze, einen an die Pflanze angrenzenden Bereich, Boden, der dazu geeignet ist, das Wachstum der Pflanze zu fördern, eine Wurzel der Pflanze, Blattwerk der Pflanze und einen Samen, der dazu geeignet ist, die Pflanze zu bilden, angewandt wird.

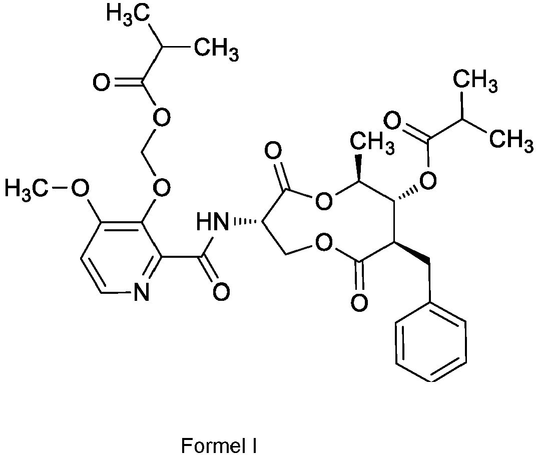

2. Das Verfahren gemäß Anspruch 1, wobei die Formulierung des Weiteren einen landwirtschaftlich akzeptablen Hilfsstoff oder Trägerstoff umfasst.

3. Das Verfahren gemäß Anspruch 1 oder Anspruch 2, wobei die Formulierung des Weiteren Azoxystrobin umfasst.

4. Das Verfahren gemäß Anspruch 1 oder Anspruch 2, wobei die Formulierung des Weiteren Tebuconazol umfasst.

5. Das Verfahren gemäß Anspruch 1 oder Anspruch 2, wobei die Formulierung des Weiteren Prothioconazol umfasst.

6. Das Verfahren gemäß Anspruch 1, umfassend: Anwenden einer fungizid wirksamen Menge der Verbindung gemäß Formel I und mindestens eines Fungizids ausgewählt aus der Gruppe bestehend aus Azoxystrobin, Tebuconazol und Prothioconazol.

## Revendications

1. Procédé de lutte et de prévention contre la moisissure blanche chez un végétal, lequel procédé comporte l'application d'une quantité à effet fongicide d'une formulation comprenant du composé de formule I, laquelle quantité efficace est appliquée sur au moins l'un des objets suivants : le végétal lui-même, une zone adjacente au végétal, un sol adapté pour soutenir la croissance du végétal, une racine du végétal, le feuillage du végétal, et une semence adaptée pour produire le végétal.

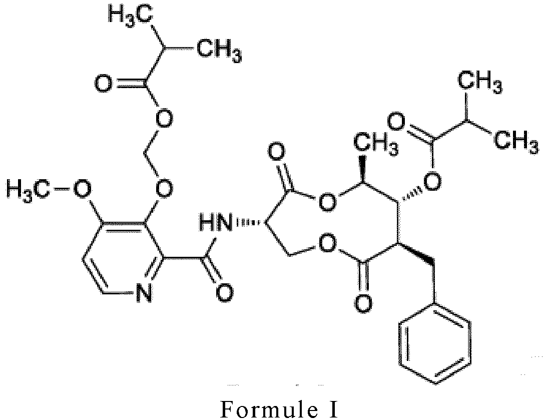

2. Procédé conforme à la revendication 1, dans lequel la formulation comprend en outre un adjuvant ou véhicule admissible en agriculture.

3. Procédé conforme à la revendication 1 ou 2, dans lequel la formulation comprend en outre de l'azoxystrobine.

4. Procédé conforme à la revendication 1 ou 2, dans lequel la formulation comprend en outre du tébuconazole.

5. Procédé conforme à la revendication 1 ou 2, dans lequel la formulation comprend en outre du prothioconazole.

6. Procédé conforme à la revendication 1, qui comporte l'application d'une quantité à effet fongicide du composé de formule I et d'au moins un fongicide choisi dans l'ensemble formé par l'azoxystrobine, le tébuconazole et le prothioconazole.
